# FASCICULE DE BREVET EUROPEEN

(11) **EP 0 934 416 B1**
(45) Date de publication et mention de la délivrance du brevet: **21.06.2006**
(21) Numéro de dépôt: 97933746.6
(22) Date de dépôt: 15.07.1997
(51) Int. Cl.: C12N 15/48, C12N 15/35, C12N 15/50, C12N 15/38, C12N 15/40, C12N 15/49, C12N 15/47, A61K 39/295

(54) **FORMULE DE VACCIN POLYNUCLEOTIDIQUE FELIN**
ZUSAMMENSETZUNG EINES POLYNUKLEOTID-IMPFSTOFFES FÜR KATZEN
FELINE POLYNUCLEOTIDE VACCINE FORMULA

(30) Priorité: 19.07.1996 FR 9609337
(43) Date de publication de la demande: 11.08.1999
(73) Titulaire: MERIAL, 69002 Lyon (FR)
(72) Inventeur: AUDONNET, Jean-Christophe, F-69006 Lyon (FR); BOUCHARDON, Annabelle, F-69007 Lyon (FR); BAUDU, Philippe, F-69290 Craponne (FR); RIVIERE, Michel, F-69130 Ecully (FR)
(74) Mandataire: Nargolwalla, Cyra
(86) Numéro de dépôt international: PCT/FR1997/001315
(87) Numéro de publication internationale: WO 1998/003660

(56) Documents cités:
- WO-A-95/20660
- WO-A-96/06934
- WARDLEY R C ET AL: "THE USE OF FELINE HERPESVIRUS AND BACULOVIRUS AS VACCINE VECTORS FOR THE GAG AND ENV GENES OF FELINE LEUKAEMIA VIRUS" JOURNAL OF GENERAL VIROLOGY, vol. 73, no. PART 07, 1 juillet 1992, pages 1811-1818, XP000288657

## Description

La présente invention a trait à une formule de vaccin permettant la vaccination des chats contre un certain nombre de pathologies. Elle a également trait à une méthode de vaccination correspondante.

On a déjà proposé par le passé des associations de vaccins contre certains virus canins.

Les associations développées jusqu'à présent étaient réalisées à partir de vaccins inactivés ou de vaccins vivants et éventuellement de mélanges de tels vaccins. Leur mise en oeuvre pose des problèmes de compatibilité entre valences et de stabilité. Il faut en effet assurer à la fois la compatibilité encre les différences valences de vaccin, que ce soit au plan des différents antigènes utilisés ou au plan des formulations elles-mêmes, notamment dans le cas où l'on combine à la fois des vaccins inactivés et des vaccins vivants. Il se pose également le problème de la conservation de tels vaccins combinés et de leur innocuité notamment en présence d'adjuvant. Ces vaccins sont en général assez coûteux.

Les demandes de brevet WO-A-90 11092. WO-A-93 19183, WO-A-94 21797 et WO-A-95 20660 ont fait usage de la technique récemment développée des vaccins polynucléotidiques. On sait que ces vaccins utilisent un plasmide apte à exprimer dans les cellules de l'hôte l'antigène inséré dans le plasmide. Toutes les voies d'administration ont été proposées (intrapéritonéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al., Nature 356, 152-154, 1992) et les injecteurs par jet liquide permettant de transfecter à la fois dans la peau, le muscle les tissus graisseux et les tissus mammaires (Furth et al., Analytical Biochemistry, 205. 365-368, 1992).

Les vaccins polynucléotidiques peuvent utiliser aussi bien des ADN nus que des ADN formulés par exemple au sein de liposomes ou de lipides cationiques.

Un objectif de l'invention est de fournir un tel vaccin qui soit de mise en oeuvre aisée et peu coûteuse.

Un autre objectif, encore de l'invention est de fournir une méthode de vaccination des chats qui permette d'obtenir une protection, avec un niveau élevé d'efficacité et de longue durée, et avec une bonne innocuité.

La présente invention a donc pour objet une formule de vaccin destiné au chat comprenant un plasmide intégrant, de manière à l'exprimer in vivo dans les cellules hôtes, un gène de pathogène félin, le virus de la leucémie féline (FeLV), le plasmide comprenant, un ou plusieurs des gènes choisis parmi le groupe consistant en env et gag/pol pour la leucémie féline.

Par gène d'agent, pathogène, on entend non seulement le gène complet, mais aussi les séquences nucléotidiques différentes, y compris fragments, conservant la capacité à induire une réponse protectrice. La notion de gène recouvre les séquences nucléotidiques équivalentes à celles décrites précisément dans tes exemples, c'est-à-dire les séquences différences mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléocidiques qui ont été modifiées pour faciliter l'expression in vivo par l'animal hôte mais codant pour la même protéine.

Pour la leucémie féline, on utilise de préférence les deux gènes env et gag/pol incégrés dans deux plasmides différents ou dans un seul et même plasmide ou la gène en seul. La présente invention concerne un vaccin tel que défini à la revendication 1.

La formule de vaccin selon l'invention pourra se présenter sous un volume de dose compris entre 0.1 et 3 ml et en particulier encre 0,5 et L ml.

La dose sera généralement comprise entre 10 ng et 1 ng, de préférence entre 100 ng et 500 µg et plus préférentiellement encore entre 1 µg et 250 µg par type de plasmide.

On utilisera de préférence des plasmides nus, simplement placés dans le véhicule de vaccination qui sera en général de l'eau physiologique (NaCl 0,9 %), de l'eau ulcrapure, du tampon TE, etc. On peut bien entendu utiliser toutes les formes de vaccin polynueléotidique décrites dans l'art antérieur.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origine humaine ou murine, ou encore éventuellement d'une autre origine telle que rat, cochon, cobaye.

De manière plus générale, la promoteur pourra être soit d'origine virale, soit d'origine cellulaire. Comme promoteur viral, on peut citer le promoteur précoce ou tardif du virus SV40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel que par exemple le promote, la desmine (Bolmont et al., Journal of Submicroscopic Cytology and Pathology, 1990, 22, 117-122 ; et ZHEMLIN et al., Gene, 1989, 78, 243-254), ou encore le promoteur de l'actine.

Lorsque plusieurs gènes sont présents dans le même plasmide, ceux-ci peuvent être présentés dans la même unité de transcription ou dans deux unités différentes.

L'invention a encore pour objet des formules de vaccin monovalent comprenant un ou plusieurs plasmides codant pour un ou plusieurs gènes de l'un des virus ci-dessus, les gènes étant ceux décrits plus haut. En dehors de leur caractère monovalent, ces formules peuvent reprendre les caractéristiques énoncées plus haut en ce qui concerne le choix des gènes, leurs combinaisons, la composition des plasmides, les volumes de dose, les doses, etc.

Les formules de vaccin monovalent peuvent aussi être utilisées (i) pour la préparation d'une formule de vaccin polyvalant tel que décrit plus haut, (ii) à titre individuel contre la pathologie propre, (iii) associées à un vaccin d'un autre type (entier vivant ou inactivé, recombinant, sous-unité) contre une autre pathologie ou (iv) comme rappel d'un vaccin comme décrit ci-après.

La présente invention a en effet encore pour objet l'utilisation d'un ou de plusieurs plasmides selon l'invention pour la fabrication d'un vaccin destiné à vacciner les chats primo-vaccinés au moyen d'un premier vaccin classique (monovalent ou multi-valent) du type de ceux de l'art antérieur, choisi notamment dans la groupe consistant en vaccin entier vivant, vaccin entier inactivé, vaccin de sous-unité, vaccin recombinant, ce premier vaccin présentant: (c'est-à-dire contenant ou pouvant exprimer) le ou les antigène(s) cadè(s) par le ou les plasmides ou antigène(s) assurant une protection croisée.

De manière remarquable, le vaccin polynucléotidique a un effet de rappel puissant se traduisant par une amplification de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

L'invention a aussi pour objet un kit de vaccination regroupant un vaccin de primo-vaccination tel que décrit ci-dessus et une formule de vaccin selon l'invention pour la rappel. Elle a aussi trait à une formule de vaccin selon l'invention accompagnée d'une notice indiquant L'usage de cette formule comme rappel d'une primo-vaccination telle que décrite ci-avant.

La présence invention a également pour objet une méthode de vaccination des chats, comprenant l'administration d'une formule de vaccin efficace telle que décrit plus haut. Cette méthode de vaccination comprend l'administration d'une ou de plusieurs doses de formulé de vaccin, ces doses pouvant être administrées successivement clans un court laps de temps et/ou successivement à des moments éloignés l'un de l'autre.

Les formules de vaccin selon l'invention pourront être administrées, dans le cadre de cette méthode de vaccination, par les différentes voies d'administration proposées dans l'art antérieur pour la vaccination polynucléotidique et au moyen des techniques d'administration connues.

L'invention a encore pour objet la méthode de vaccination consistant à faire une primo-vaccination telle que décrite ci-dessus et un rappel avec une formule de vaccin selon l'invention.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 6 semaines, on assure l'administration du vaccin polyvalent, ou monovalent selon l'invention.

L'efficacité de la présentation des antigènes au système immunitaire varie en fonction des tissus. En particulier, les muqueuses de l'arbre respiratoire servent de barrière à l'entrée des pathogènes et sont associées à des tissus lymphoides qui supportent une immunité locale. L'administration d'un vaccin par contact avec les muqueuses, en particulier muqueuses buccale, pharyngée et région bronchique présente un intérêt certain pour la vaccination contre les patholoqies respiratoires et digestives.

En conséquence, les voies d'administration mucosales font partie d'un mode d'administration préféré pour l'invention, utilisant notamment la nébulisation ou spray ou l'eau de boisson. Les formules de vaccin et méthodes de vaccination selon l'invention pourront être appliquées dans ce cadre.

L' invention concerne aussi la méthode de préparation des formules de vaccin, à savoir la préparation des valences et leurs mélanges, telle qu'elle ressort de cette description.

L'invention va être maintenant décrite plus en détails à l'aide de modes de réailsation de l'invention pris en référence aux cessins annexés.

### Liste des figures

- Figure N° 1 :: Plasmide pVR1012
- Figure N° 2 :: Plasmide pP8179
- Figure N° 3 :: Séquence du gène env FeLV-B
- Figure N° 4 :: Plasmide pPB180
- Figure N° 5 :: Séquence gag/pol du virus FeLV-A souche Glasgow-1
- Figure N° 6 :: Plasmide pPB181

### Liste des séquences SEQ ID N°

- SEO ID N° 1 :: Oligonucléotide PB247
- SEQ ID N° 2 :: Oligonuctéotide PB249
- SEQ ID N° 3 :: Oligonucléotide PB281
- SEQ ID N° 4 :: Oligonucléotide PB282
- SEQ ID N° 5 :: Séquence du gène env du virus FeLV-B
- SEQ ID N° 6 :: Oligonucléotide PB283
- SEQ ID N° 7 :: Oligonucléotide PB284
- SEQ ID N° 8 :: Séquence du gène gag/pol du virus FeLV-A (Glasgow-1)

### EXEMPLES

### Exemple 1 : Culture des virus

Les virus sont cultivés sur le système cellulaire approprié jusqu' à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Example 2 : Extraction des ADNs génomiques viraux:

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrés est traitée par la protéinase K (100 *µ*g/ml final) en présence de sodium dodecyl sulfate (SDS) (0.5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 3 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénolchloroforme" décrite par P. Chomczynski et N. Sacchi (Anal. Biochem. 1987, 162, 156-159).

### Exemple 4 : Techniques de biologie moléculaire

Toutes les constructions de plasmides ont été réalisées en utilisant les techniques standards de biologie moléculaire décrites par J. Sarnbrook *et al.,* (*Molecular Cloning: A Laboratory Manual.* 2^{nd} Edition, Cold Spring Harbor Laboratory. Cold Spring Harbor. New York, 1989). Tous les fragments de restriction utilisés pour la présente invention ont été isolés en utilisant le kit "Geneclean" (810101 Inc. La Jolla, CA).

### Exemple 5 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaine par polymérase (PCR) ont été effectuées selon les techniques standards (Sambrook J. *et al.* 1989). Chaque réaction de RT-PCR a été faite avec un couple d'amplirners spécifiques et en prenant comme matrice l'ARN génomique viral extrait. L'ADN complémentaire amplifié a été extrait au phénol/chloroforme/alcoolisoamylique (25:24:1) avant d'être digéré par les enzymes de restriction.

### Exemple 6: plasmide pVR1012

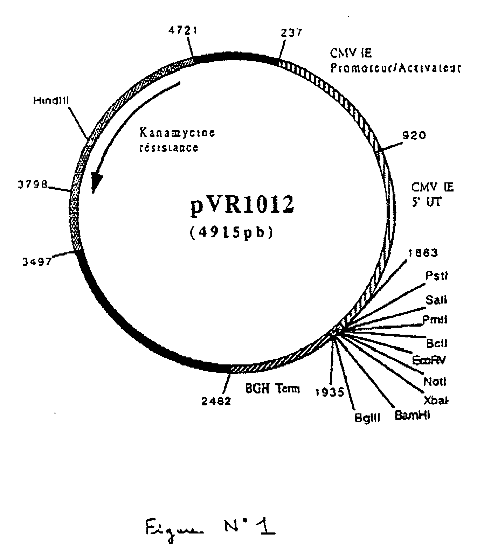

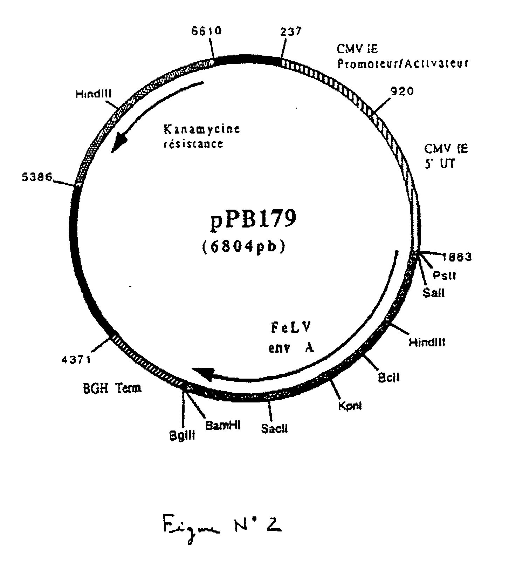

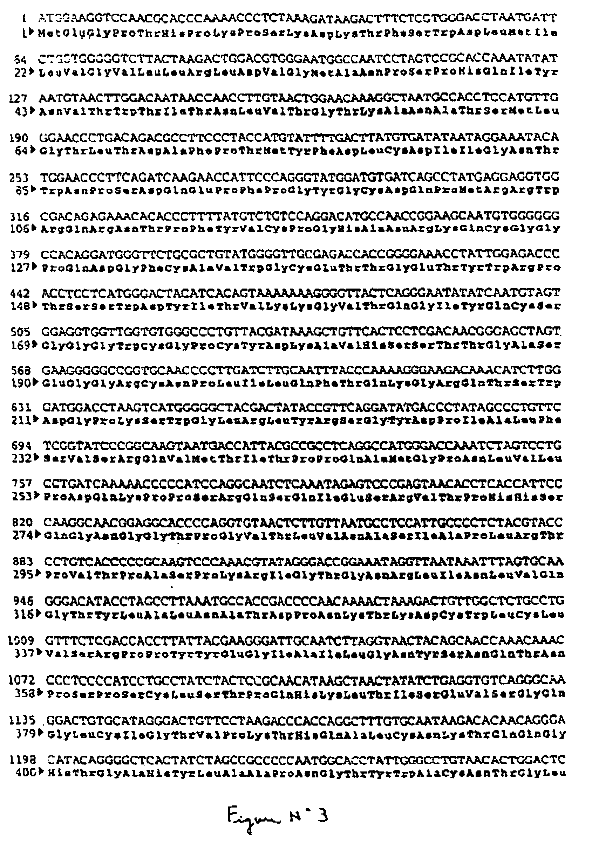

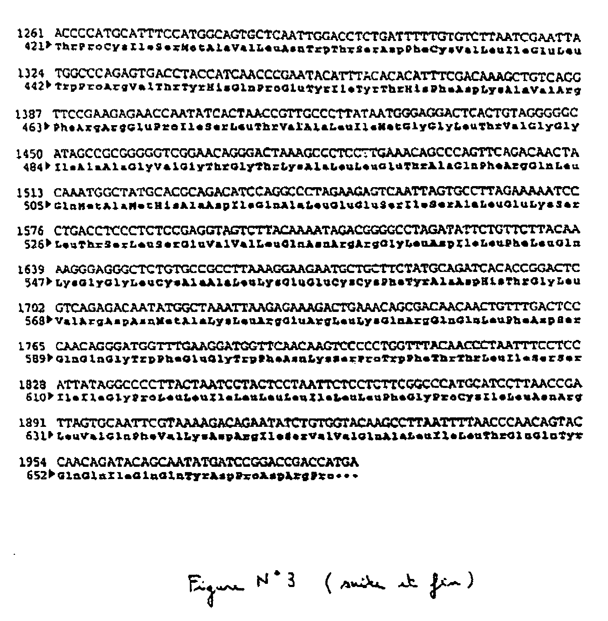

Le plasmide pVR1012 (Figure N° 1) a été obtenu auprès de Vical inc. San Diego, CA, USA, Sa construction a été décrite dans J. Hartikka *et al.* (Human Gene Therapy, 1996, 7, 1205-1217).

### Exemple 7 : Construction du plasmide pPB179 (gène env virus FeLV-A)

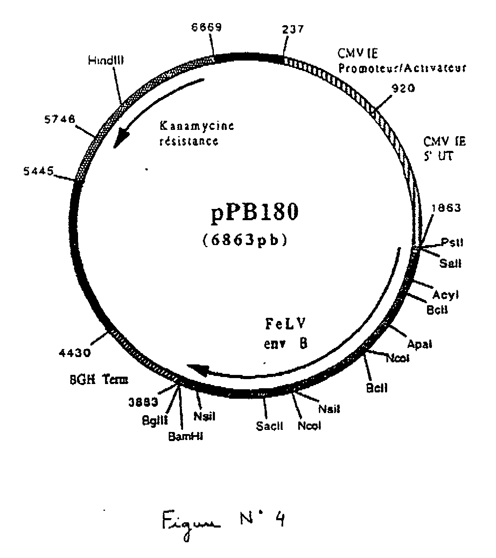

Une réaction de RT-PCR selon la technique de l'exemple 5 a été réalisée avec l'ARN génomique du virus de la leucémie féline (FeLV-A) (Souche Glasgow-1) (M. Stewart *et al.* J. Virot, 1986, 58, 825-834), préparé selon la technique de l'exemple 3, et avec les oligonucléotides suivants:
P8247 (29 mer) (SEQ ID N° 1)
   5'TTTGTCGACCATGGAAAGTCCAACGCACC 3'
PB249 (28 mer) (SEQ ID N° 2)
   5'TTTGGATCCTCATGGTCGGTCCGGATCG 3'
pour amplifier un fragment de 1947 pb contenant le gène codant pour la glycoprotéine Env du virus FeLV.A (souche Glasgow-1) sous la forme d'un fragment Sali-SamHl. Après purification, le produit de RT-PCR a été digéré par *Sal*I et *Bam*HI pour donner un fragment SalI-BamHI de 1935 pb,
Ce fragment a été ligaturé avec le vecteur pVR1012 (exemple 6), préalablement digéré avec *Sal*I et *Bam*HI, pour donner la plasmide pP8179 (6804 pb) (Figure N° 2).

### Exemple 8 : Construction du plasmide pPB180 (gène env virus FeLV-B)

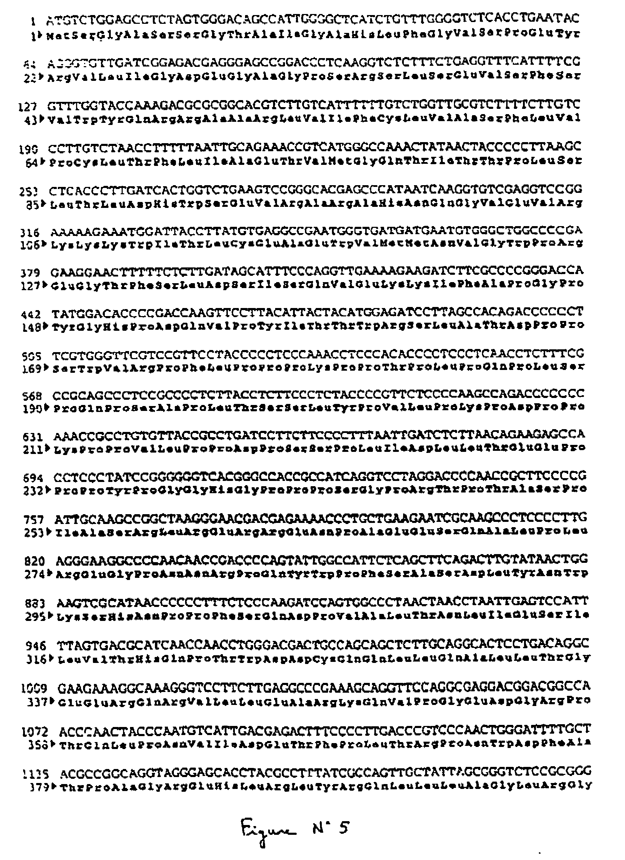

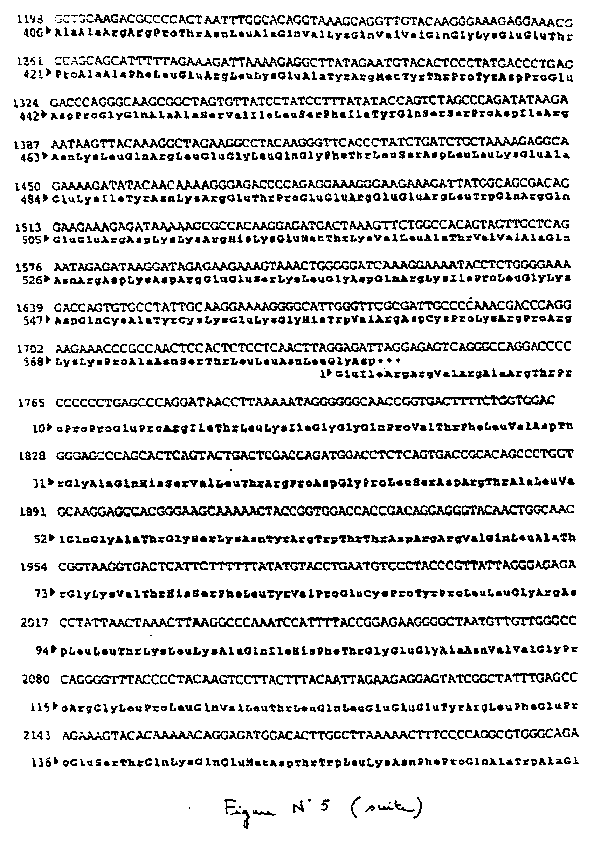

Une réaction de RT-PCR selon la technique de l'exemple 5 a été réalisée avec l'ARN génomique du virus de la leucémie féline (sous-type FeLV-B), préparé selon la technique de l'exemple 3, et avec les oligonucléotides suivants:
PB281 (29 mer) (SEQ ID N° 3)
   5'TTTGTCGACATGGAAGCTCCAACGCACCC 3'
PB282 (32 mer) (SEQ ID N° 4)
   5'TTGGATCCTCATGGTCGGTCCGGATCATATTG 3'
pour amplifier un fragment de 2005 pb contenant le gène codant pour ta glycoprotéine Env du virus FeLV-B (Figure N° 3 et SEQ ID N° 5) sous la forme d'un fragment SalI.BamHI. Après purification, le produit de RT-PCR a été digéré par *Sal*I et *Bam*HI pour donner un fragment SalI-BamHI de 1995 pb.
Ce fragment a été ligaturé avec le vecteur pVR1012 (exemple 6), préalablement digéré avec *Sal*I et *Bam*HI, pour donner le plasmide pPB180 (6863 pb) (Figure N° 4).

### Exemple 9 : Construction du plasmide pP8181 (gène FeLV gag/pol)

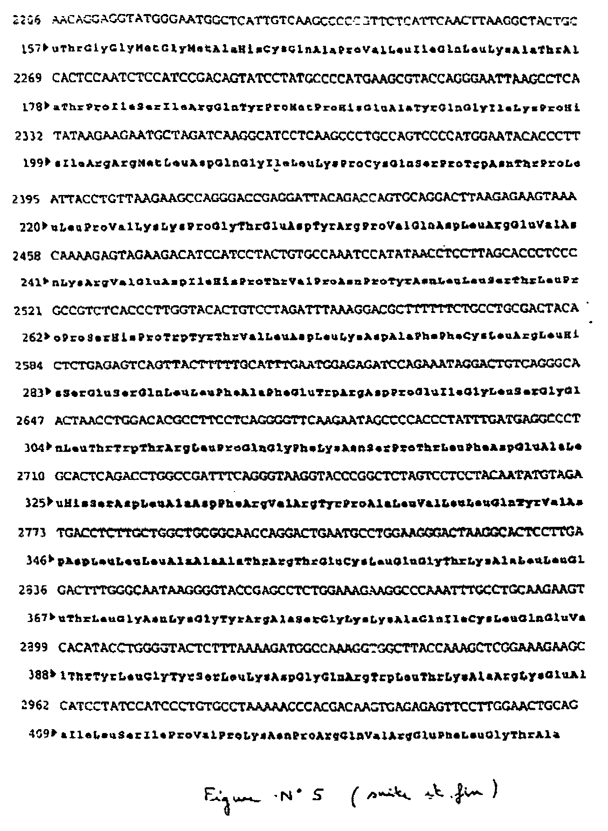

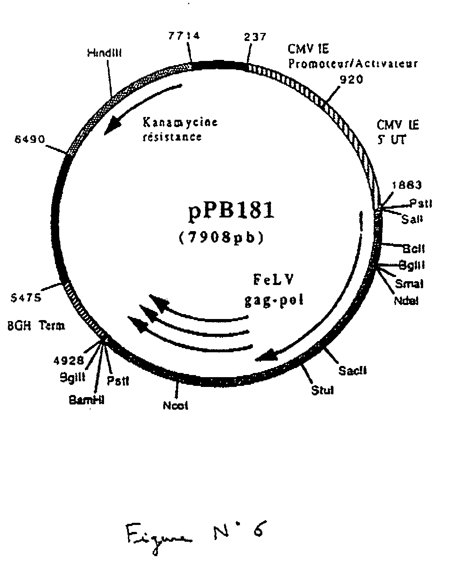

Une réaction de RT-PCR selon la technique de l'exemple 5 a été révisée avec l'ARN génomique du virus de la leucémie féline (sous-type FeLV-A) (Souche Glasgow-1), préparé selon la technique de l'exemple 3, et avec les oligonucléotides suivants:
PB283 (33 mer) (SEQ ID N° 6)
   5'TTGGATCCTTATTTAATTACTGCAGTTCCAAGGAACTCTC 3'
PS284 (40 mer) (SEQ ID N° 7)
   5'TTGGATCCTTATTTAATTACTGCAGTTCCAAGGAACTCTC 3'
pour amplifier un fragment de 3049 pb contenant la séquence codant pour la protéine Gag et la partie 5' de la séquence codant pour la protéine Pol du virus FeLV-A (souche Glasgow-1) (Figure N° 5 et SEQ ID N° 3) sous la forme d'un fragment SalI-BamHI. Après purification, le produit de RT-PCR a été digéré par *Sal*I et *Bam*HI pour donner un fragment SalI-BamHI de 3039 pb.
Ce fragment a été ligaturé avec le vecteur pVR1012 (exemple 6), préalablement digéré avec *Sal*I et *Bam*HI, pour donner le plasmide pPB181 (7908 pb) (Figure N° 6).

### Exemple 10: Production et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook *et al.* (*Molecular Cloning: A Laboratory Manual.* 2^{nd} Edition. Cold Spring Harbor Laboratory. Cold Spring Harbor, New York, 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins (voir exemple 11), les plasmides purifiés sont resuspendus de manière à obtenir des solutions à haute concentration (> 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 11: Fabrication des vaccins associés

Les divers plasmides nécessaires à la fabrication d'un vaccin associé sont mélangés à partir de leurs solutions concentrées (exemple 10). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACl à 0,9 %, soit du tampon pas.
Des formulations particulières telles que les liposomes, les lipides cationiques, peuvent aussi être mises en oeuvre pour la fabrication des vaccins.

### Exempte 12: Vaccination des chats

les chats sont vaccinés avec des doses de 10 *µ*g, 50 µg ou 250 µg par plasmide.
Les injections sont réalisées à l'aiguille par voie intramusculaire. Dans ce cas, les doses vaccinales sont administrées sous un volume de 1 ml.
Les injections peuvent aussi être réalisées à l'aiguille par voie intradermique. Dans ce cas, les doses vaccinales sont administrées sous un volume total de 1 ml administré en 10 points de 0,1 ml ou en 20 points de 0,05 ml. Les administrations intradermiques sont réalisées après avoir rasé la peau (flanc thoracique en général) ou bien au niveau d'une région anatomique relativement glabre, par exemple la face interne de la cuisse.
On peut aussi utiliser un appareil d'injection à jet liquide (sans aiguille) pour les injections intradermiques.

## Revendications

1. Vaccin pour chat comprenant un plasmide contenant et exprimant *in vivo* un ou plusieurs acides nucléiques codant pour env et/ou gag/pol du virus de la leucémie féline, et un véhicule approprié.

2. Le vaccin selon la revendication 1, **caractérisé en ce que** ledit plasmide contient et exprime *in vivo* un acide nucléique codant pour env.

3. Le vaccin selon la revendication 1, **caractérisé en ce que** ledit plasmide contient et exprime *in vivo* un acide nucléique codant pour env et gal/pol.

4. Le vaccin selon la revendication 1, **caractérisé en ce que** ledit plasmide contient et exprime *in vivo* un acide nucléique codant pour env et comprend un autre plasmide contenant et exprimant *in vivo* un acide nucléique codant pour gal/pol.

5. Le vaccin selon l'une quelconque des revendications 1 à 4, **caractérisé en ce que** le plasmide comprend un promoteur choisi parmi : le promoteur CMV-IE, le promoteur précoce du virus SV40, le promoteur tardif du virus SV40, le promoteur LTR du virus du sarcome de Rous et le promoteur d'un gène du cytosquelette.

6. Le vaccin selon la revendication 5, **caractérisé en ce que** ledit plasmide contient un promoteur CMV-IE.

7. Le vaccin selon la revendication 5, **caractérisé en ce que**, le promoteur d'un gène du cytosquelette est le promoteur de la desmine ou le promoteur de l'actine.

8. Le vaccin selon l'une quelconque des revendications 1 à 7, comprenant le 10 ng à 1 mg, de préférence de 100 ng à 500 µg, et plus préférentiellement de 1 µg à 250 µg de chaque plasmide.

9. Utilisation de un ou plusieurs plasmides tels que décrits dans l'une quelconque des revendications 1 à 7 pour la fabrication d'un vaccin destiné à vacciner un chat primo-vacciné au moyen d'un premier vaccin sélectionné dans le groupe consistant en un vaccin entier, un vaccin entier inactivé, un vaccin de sous-unité, un vaccin recombinant, ce premier vaccin ayant le ou les antigène(s) codé(s) par le ou les plasmide(s) ou antigène(s) assurant une protection croisée.

10. Un kit de vaccination pour chat regroupant un vaccin selon l'une quelconque des revendications 1 à 8, et un autre vaccin pour chat sélectionné dans le groupe consistant en un vaccin anti-inactivé, un vaccin de sous-unité, un vaccin recombinant, ledit autre vaccin ayant l'antigène codé par le plasmide du vaccin ou un antigène assurant une protection croisée, pour l'administration dudit autre vaccin en primo-vaccination suivie de l'administration du vaccin contenant le plasmide en rappel.

11. utilisation d'un ou de plusieurs plasmides tel que décrit dans l'une quelconque des revendications 1 à 7, pour la fabrication d'un vaccin destiné à la vaccination d'un chat en combinaison avec un vaccin anti-inactivé, un vaccin entier vivant, un vaccin recombinant et un vaccin de sous-unité contre une autre maladie féline.

## Claims

1. A vaccine for cats comprising a plasmid containing and expressing in vivo one or more nucleic acids coding for env and/or gag/pol of the virus of feline leukaemia, and a suitable carrier.

2. The vaccine according to claim 1 **characterised in that** said plasmid contains and expresses in vivo a nucleic acid coding for env.

3. The vaccine according to claim 1 **characterised in that** said plasmid contains and expresses in vivo a nucleic acid coding for env and gal/pol.

4. The vaccine according to claim 1 **characterised in that** said plasmid contains and expresses in vivo a nucleic acid coding for env and comprises another plasmid containing and expressing in vivo a nucleic acid coding for gal/pol.

5. The vaccine according to any one of claims 1 to 4 **characterised in that** the plasmid comprises a promoter selected from: the CMV-IE promoter, the SV40 virus early promoter, the SV40 virus late promoter, the Rous sarcoma virus LTR promoter and the promoter of a gene of the cytoskeleton.

6. The vaccine according to claim 5 **characterised in that** said plasmid contains a CMV-IE promoter.

7. The vaccine according to claim 5 **characterised in that** the promoter of a gene of the cytoskeleton is the promoter of desmin or the promoter of actin.

8. The vaccine according to any one of claims 1 to 7 comprising 10 ng to 1 mg, preferably from 100 ng to 500 µg and more preferably from 1 µg to 250 µg of each plasmid.

9. Use of one or more plasmids as described in any one of claims 1 to 7 for the production of a vaccine intended to vaccinate a cat primo-vaccinated by means of a first vaccine selected from the group consisting of a whole vaccine, an inactivated whole vaccine, a subunit vaccine, a recombinant vaccine, said first vaccine having the antigen(s) coded by the plasmid(s) or antigen(s) providing cross-protection.

10. A vaccination kit for cats comprising a vaccine according to any one of claims 1 to 8 and another vaccine for cats selected from the group consisting of an anti-inactivated vaccine, a subunit vaccine, a recombinant vaccine, said other vaccine having the antigen coded by the plasmid of the vaccine or an antigen providing cross-protection, for the administration of said other vaccine in primo-vaccination followed by administration of the vaccine containing the plasmid as a booster.

11. Use of one or more plasmids as described in any one of claims 1 to 7 for the production of a vaccine intended for the vaccination of a cat in combination with an anti-inactivated vaccine, a live whole vaccine, a recombinant vaccine and a subunit vaccine against another feline disease.

## Patentansprüche

1. Impfstoff für Katzen, der ein Plasmid, das eine oder mehrere Nucleinsäuren, die env und/oder gag/pol des felinen Leukämievirus codieren, enthält und *in vivo* exprimiert, und geeignete Träger umfasst.

2. Impfstoff nach Anspruch 1, **dadurch gekennzeichnet, dass** das Plasmid eine Nucleinsäure enthält und *in vivo* exprimiert, die env codiert.

3. Impfstoff nach Anspruch 1, **dadurch gekennzeichnet, dass** das Plasmid eine Nucleinsäure enthält und *in vivo* exprimiert, die env und gal/pol codiert.

4. Impfstoff nach Anspruch 1, **dadurch gekennzeichnet, dass** das Plasmid eine Nucleinsäure enthält und *in vivo* exprimiert, die env codiert, und ein anderes Plasmid umfasst, das eine Nucleinsäure enthält und *in vivo* exprimiert, die gal/pol exprimiert.

5. Impfstoff nach einem der Ansprüche 1 bis 4, **dadurch gekennzeichnet, dass** das Plasmid einen Promotor umfasst, der ausgewählt ist aus dem CMV-IE-Promotor, dem frühen SV40 Virus-Promotor, dem späten SV40 Virus-Promotor, dem LTR-Promotor des Rous-Sarcom-Virus und dem Promotor eines Gens des Cytoskeletts.

6. Impfstoff nach Anspruch 5, **dadurch gekennzeichnet, dass** das Plasmid einen CMV-IE-Promotor enthält.

7. Impfstoff nach Anspruch 5, **dadurch gekennzeichnet, dass** der Promotor eines Gens des Cytoskeletts der Desminpromotor oder der Aktinpromotor ist.

8. Impfstoff nach einem der Ansprüche 1 bis 7, umfassend 10 ng bis 1 mg, vorzugsweise 100 ng bis 500 µg und mehr bevorzugt 1 µg bis 250 µg jedes Plasmids.

9. Verwendung eines oder mehrerer Plasmide, wie in einem der Ansprüche 1 bis 7 beschrieben, zur Herstellung eines Impfstoffes, der bestimmt ist zur Impfung einer erstgeimpften Katze mittels eines ersten Impfstoffes, der ausgewählt ist aus der Gruppe bestehend aus: einem Vollimpfstoff, einem inaktivierten Vollimpfstoff, einem Untereinheiten-Impfstoff, einem rekombinanten Impfstoff, wobei der erste Impfstoff das oder die durch das oder die Plasmid(e) codierte(n) Antigen(e) umfasst oder Antigen(e), das (die) einen Kreuzschutz sicherstellt (sicherstellen).

10. Kit zur Impfung von Katzen, der einen Impfstoff nach einem der Ansprüche 1 bis 8 und einen weiteren Impfstoff für Katzen umfasst, der ausgewählt ist aus der Gruppe bestehend aus: einem anti-inaktivierten Impfstoff, einem Untereinheiten-Impfstoff, einem rekombinanten Impfstoff, wobei der weitere Impfstoff das durch das Plasmid des Impfstoffes codierte Antigen umfasst oder ein Antigen, das einen Kreuzschutz sicherstellt, zur Verabreichung des weiteren Impfstoffes als Erstimpfung gefolgt von der Verabreichung des das Plasmid enthaltenden Impfstoffes zur Auffrischung.

11. Verwendung eines oder mehrerer Plasmide, wie in einem der Ansprüche 1 bis 7 beschrieben, zur Herstellung eines Impfstoffes, der bestimmt ist zur Impfung einer Katze in Kombination mit einem anti-inaktivierten Impfstoff, einem Lebendvollimpfstoff, einem rekombinanten Impfstoff und einem Untereinheiten-Impfstoff gegen eine andere feline Erkrankung.
